# EUROPEAN PATENT SPECIFICATION

(11) **EP 4 159 260 B1**
(45) Date of publication and mention of the grant of the patent: **28.01.2026**
(21) Application number: 21818974.4
(22) Date of filing: 27.05.2021
(51) Int. Cl.: A61K 33/00, A61M 16/00, A61M 16/12, A61K 9/00, A61P 9/00

(54) **ARTICIFICAL GAS MIXTURE FOR THE EMERGENCY MANAGEMENT OF ACUTE ISCHEMIC ATTACKS WITH IMPAIRED CEREBRAL OR CORONARY CIRCULATION**
KÜNSTLICHES GASGEMISCH ZUR NOTFALLBEHANDLUNG AKUTER ISCHÄMISCHER ATTACKEN MIT BEEINTRÄCHTIGTEM HIRN- ODER KORONARKREISLAUF
MÉLANGE GAZEUX ARTIFICIEL POUR LA GESTION D'URGENCE D'ACCIDENTS ISCHÉMIQUES AIGUS AVEC UNE CIRCULATION CÉRÉBRALE OU CORONARIENNE ALTÉRÉE

(30) Priority: 01.06.2020 RU 2020119170
(43) Date of publication of application: 05.04.2023
(73) Proprietor: Petrov, Vasilii Aleksandrovich, Saint-Petersburg, poselok Levashove, 194361 (RU); Ivanov, Andrey Olegovich, Saint-Petersburg, 192012 (RU); Kindzerskiy, Anatoly Valerievich, Saint-Petersburg, 197110 (RU); Mayorov, Ivan Viktorovich, Saint-Petersburg, 194064 (RU)
(72) Inventor: Petrov, Vasilii Aleksandrovich, Saint-Petersburg, poselok Levashove, 194361 (RU); Ivanov, Andrey Olegovich, Saint-Petersburg, 192012 (RU); Kindzerskiy, Anatoly Valerievich, Saint-Petersburg, 197110 (RU); Mayorov, Ivan Viktorovich, Saint-Petersburg, 194064 (RU)
(74) Representative: Beck & Rössig European Patent Attorneys
(86) International application number: PCT/RU2021/050140
(87) International publication number: WO 2021/246917

(56) References cited:
- WO-A1-2019/008014
- RU-C2- 2 661 771
- RU-C2- 2 684 748
- RU-C2- 2 684 748
- JAWAD N ET AL: "Neuroprotection (and lack of neuroprotection) afforded by a series of noble gases in an in vitro model of neuronal injury", NEUROSCIENCE LETTERS, ELSEVIER, AMSTERDAM, NL, vol. 460, no. 3, 4 September 2009 (2009-09-04), pages 232 - 236, XP026219307, ISSN: 0304-3940, [retrieved on 20090607], DOI: 10.1016/J.NEULET.2009.05.069
- ANANEV V.N.: "Mekhanizmy gipobioza pri dykhanii gazovymi smesyami s argonom, kriptonom i ksenonom [MECHANISMS HIBERNATION WHILE BREATHING GAS MIXTURES WITH ARGON, KRYPTON AND XENON]", SOVREMENNYE PROBLEMY NAUKI I OBRAZOVANIYA [MODERN PROBLEMS OF SCIENCE AND EDUCATION], no. 4, 2015, pages 1 - 9, XP009533110, ISSN: 2070-7428

## Description

### FIELD OF THE INVENTION

The invention relates to the field of medicine, in particular, to primary (premedical and medical) health care, emergency medical services, including emergency specialized medical care, and is intended to stop and inhibit the development of the acute phase of ischemic stroke and coronary insufficiency, mainly before the arrival of the ambulance. More specifically, the present invention relates to medicinal product for use by inhalation in the emergency management of acute ischemic attacks with impaired cerebral or coronary circulation, which is an artificial gas mixture including oxygen and argon.

### BACKGROUND ART

The procedure, methods, and means of providing specialized medical care to patients with stroke are known from the state of art (Order of the Ministry of Health of the Russian Federation No. 513 of August 1, 2007 "On approval of the standard of medical care for patients with stroke (when providing specialized care)").

There are also many recommendations and guidelines for the general public on how to provide medical care for strokes and heart attacks in the premedical phase. These instructions can be found in school first aid courses, medical and non-medical schools, and the media. These instructions suggest the simplest measures that can do no harm to the patient, but in some cases also measures with the use of medical procedures and drugs (see, for example, https://medbe.ru/materials/ostrye-zabolevaniya/neotlozhnaya-pomoshch-priostrykh-narusheniyakh-mozgovogo-krovoobrashcheniya/ at www.MedBe.ru and at https://doktor-ok.com, "The 8 Essentials of Stroke First Aid"),

At the same time, the standard procedure for premedical care implies:
- ensuring secure airway;
- oxygen inhalation through a nasal catheter;
- control of psychomotor agitation by intramuscular injection of 1-2 ml of 3% Phenazepam solution;
- for high blood pressure: intramuscularly 2-4 ml of 2% Papaverine solution and 2-4 ml of 1% Dibasol solution;
- for arterial hypotension: 2-4 ml Cordiamin or 2-4 ml 10% Sulfocamphocaine solution.

In general, such a scheme is aimed at preventing hypoxia of the brain and heart muscle, controlling pain shock and ensuring the preservation of vital functions until the arrival of the ambulance.

When providing primary (premedical and medical) health care before arriving at the hospital, health workers need to properly assess the patient's condition and provide adequate medical care at the scene and while transporting the patient to the hospital. Early diagnosis, timely implementation of the treatment methods established by the protocol increases the chances of survival and reduces the complications of stroke and heart attack.

Obviously, the combination of the proposed measures is difficult to implement in the domestic conditions of premedical care in the absence of specialists.

One of the main areas of specialized medical care in the acute phase of ischemic stroke and coronary insufficiency in the inpatient setting is also the provision of antihypoxic and thrombolytic therapy, the administration of antiaggregants and neuroprotectants. The most important task is to avoid prolonged ischemia of the penumbral area and expansion of the infarct area.

There are known methods of organoprotection, including non-medicinal and medicinal methods and means of influencing the patient's body.

Medicinal methods include the administration of drugs with antihypoxic, neuroprotective and nootropic effects (antihypoxants).

There is a method of nerve cell protection in acute stroke by injecting the drug Noopept (patent of the Russian Federation No. 2330680, published on 10.08.2008). According to the authors of the invention, the use of Noopept^{®} leads to a significant reduction of the area of brain lesions in experimental ischemia.

Another class of antihypoxants includes, for example, Hypoxen^{®} (sodium polydihydroxyphenylenethiosulfonate), which regulates cell metabolism and is taken orally (patent of the Russian Federation No. 2105000, published on 20.02.1998; U.S. Patent No. 6117970, published on 12.09.2000). Hypoxen^{®} restores the process of macroenergy generation disturbed or interrupted by some or other pathological processes. It is used for treatment of ischemic damage of CNS, chronic fatigue syndrome, intoxication by hypoxic poisons and other chronic and acute hypoxic conditions.

The disadvantages of medical methods of treatment at the pre-medical stage are the need for the participation of medical personnel, low effectiveness in deep or widespread lesions, the inability to use for preventive purposes, side effects, the presence of contraindications, the high cost of drugs and others.

Non-drug methods with antihypoxic effects include exposure to gas mixtures with an increased oxygen content.

There are methods of exposure to gas mixtures with increased oxygen content with antihypoxic effects.

For example, we know an effective method of hyperbaric oxygen therapy, which consists in treatment with oxygen under increased pressure (see, for example, Petrovsky B.V., Efuni S.N. Fundamentals of hyperbaric oxygenation. - M.: Meditsina, 1976, 344 p.; Artru F., Charcornac R., Deleuze R. Hyperbaric oxygenation for severe head injuries: Preliminary results of controlled study. Eur Neurol. 1976, v.14, p. 310-318; Murthy T. Role of hyperoxia and hyperbaric oxygen in severe head injury: A review. Indian Journal of Neurotrauma 2006; Vol 3; 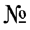 2: 77-80). The therapeutic effect of hyperbaric oxygen therapy is based on a significant increase in oxygen tension in the body's fluid media (blood, lymph, and tissue fluid), which allows rapid delivery of oxygen to tissues suffering from hypoxia and promotes restoration of cellular respiration. However, the key disadvantage of this method is possible complications, mainly in the form of increased intracranial pressure.

Another effective method of relieving the acute development of ischemic stroke is the use of oxygen antihypoxic therapy along with a lung ventilator (see Features of oxygen transport in the acute period of ischemic stroke (K.V. Lukashev et al//V.A. Netovsky Research Institute of General Resuscitation, Russian Academy of Medical Sciences, online: cyberleninka.ru, 2010).

Clearly, the methods described can be applied neither in premedical care nor by patients on their own.

There is a method of oxygen therapy of stroke, heart attack, coronary heart disease, and other diseases in their acute phase, accompanied by hypoxia, in premedical care (including self-help), implemented with the use of portable cylinders with oxygen, breathing with which is aimed at reducing hypoxic phenomena (see, for example, online: medprep.info/ oxygen; or via other online source: med.ru/ use of oxygen therapy for heart disease).

A single cylinder can provide oxygen-enriched breathing for several minutes, for example:
- Spare Air 0.28 liters, 20 MPa, air capacity at normal conditions - 56 liters, diameter 5.71 cm, length 22.23 cm, weight 0.687 kg, refillable (about 6 minutes of use);
- Spare Air 0.42 liters, 20 MPa, air capacity at normal conditions - 82 liters, diameter 5.71 cm, length 34 cm, weight 0.985 kg, refillable (about 10 minutes of use), and others.

A method is described of using the Air-Active oxygen cylinder in the form of inhalations, as stated in its description, to eliminate oxygen starvation (hypoxia), normalize metabolism in the human body, increase mental and physical performance and endurance, as well as to prepare oxygen cocktails in domestic conditions. Air-Active oxygen cylinder contains a gas mixture of Ar - 25%, O2 - 75% (produced by Tyumenskiye Aerozoli LLC).

The indicated mixture of oxygen and argon is more effective for the purpose of preventing organ hypoxia, but the content of argon in this mixture is at 25% vol.; the lack of other effective components, such as xenon, and a small stock of gas mixture, which does not allow the patient to breathe for the entire period of time, which it takes - in most cases - for the arrival of the ambulance, limit the effectiveness of this method for the purposes of acute ischemic attacks in premedical care.

Moreover, when using such breathing gas mixtures, the specificity of their pharmacokinetics and pharmacodynamics can also be noted as a disadvantage, because their distribution in the human body and interaction with cells and organs occurs during direct breathing and quickly ceases in the absence of gas mixtures due to the dynamic desaturation of body tissues.

The closest to the claimed medicinal product is the Method of auxiliary therapy in the treatment and rehabilitation of patients with disorders of the oxygen balance of the body under Russian Federation patent for invention No. 2661771, published on 19.07.2018, taken as a prototype.

According to this last method, in order to achieve a pronounced neuroprotective and organoprotective effect in relieving acute manifestations of hypoxic conditions, the formation and maintenance of artificial hyperoxic argon-containing gas medium containing argon 30-70 vol.%, oxygen - 25-70 vol.%, nitrogen - the rest, or artificial hypoxic argon-containing gas medium containing argon 30-70 vol.%, oxygen - 14-17 vol.%, nitrogen - the rest is performed in inhalation system. This method allows to increase significantly the adaptation and compensatory possibilities of the body in patients with disorders of oxygen balance.

At the same time, however, therapeutic application of the gas mixture preparation of the above composition should be performed under hospital conditions in the form of a course of cyclic respiratory effects with artificial gas mixtures, mostly daily or every other day, and a specially created chamber or other airtight room - a hospital ward, box, or tent, equipped with a system of preparation and supply of gas mixtures - should be used as an inhalation system. Obviously, these conditions limit the possibility of using this medicinal product for the purposes of acute ischemic seizures in premedical care.

RU 2684748 C2 discloses method of long-term maintenance of human vitality in the field with injuries with large blood loss, including stopping bleeding by applying dressings and harnesses, administering hemostatic, narcotic drugs, blood substitutes, wherein the wounded person is transferred to the breathing mode with an artificial gas mixture of up to 6 hours or more, containing up to 1-35% by volume xenon, 30-35% vol. argon, not less than 21% vol. oxygen and nitrogen - the rest.

WO 2019/008014 A1 discloses a pharmaceutical composition comprising a gas mixture of xenon and argon for use in a method for preventing and/or treating ischemic insults, wherein the volume proportion of xenon is between 5 and 20 %, the volume proportion of argon is between 5 and 20 %, and the pharmaceutical composition further comprises a gas complement to reach 100% volume proportion. Disclosed oxygen contents range from 20 to 50%.

### SUMMARY OF THE INVENTION

The present invention relates to a medicinal product for use by inhalation in the emergency management of acute ischaemic attacks with impaired cerebral or coronary circulation, which is an artificial gas mixture including oxygen and argon, characterized in that it contains xenon 1-10 vol.%, argon 30-35 vol.%, and oxygen 60-65 vol.%.

### DETAILED DESCRIPTION

The technical problem underlying the present invention is creating an effective, safe, simple, reliable, inexpensive medicinal product for relieving acute ischemic attacks associated with impaired cerebral or coronary circulation, reducing the risk of acute cerebral stroke and myocardial infarction, at the stages of premedical (in the absence of medical specialists and the necessary medical equipment) and primary medical care.

This technical problem is solved by the subject matter of claim 1.

The technical result from the use of the claimed invention is to increase the effectiveness, safety and speed of relieving acute cerebral and cardiac ischemia, reducing the risk and inhibition of acute cerebral stroke and myocardial infarction within 20-40 minutes or more, at least until the arrival of the ambulance, thanks to the use of a special breathing gas mixture with a simple individual device, which can be used independently or with an assistant.

Anti-ischemic use of breathing mixtures of the proposed composition is possible at the stages of primary health care, during delivery to the hospital, as well as, if necessary, during resuscitation and treatment in inpatient settings.

The specified technical result is achieved by emergency relief of acute ischemic attacks with impaired cerebral or coronary circulation through the use of respiratory gas mixtures, including respiratory exposure to the patient with an artificial gas mixture (ARGM), with an increased argon content of at least 30 vol.%, is used, which is administered by inhalation, ensuring the effect of this ARGM continuously throughout the procedure, the patient is exposed to using a personalized inhalation device, in which a breathing gas environment with increased oxygen content and addition of xenon composition is created: xenon - 1-10 vol.%, argon - 30-35 vol.%, oxygen - 60-65 vol. %.

At the same time, exposure of the patient to artificial gas mixture of the above composition is carried out mainly at the stage of premedical care, or during the delivery of the patient to the hospital, or, if necessary, already at the hospital during the treatment and resuscitation activities in patients with an acute ischemic attack and violation of the brain or coronary circulation.

In addition, exposure of the patient to an artificial gas mixture of the above composition is carried out for 20-40 minutes or more, before the provision of specialized medical care.

A face mask, respirator or mouthpiece, connected to a cylinder with oxygen-argon- xenon gas mixture of the specified composition can be used as an individual inhalation device.

The clinical effect of the use of artificial respiratory media with increased argon content, altered (increased) oxygen content, as well as xenon included in their composition, is provided by an increase in the volume of oxygen delivered to tissues and organs damaged by an acute ischemic attack with impaired cerebral or coronary circulation, acceleration of its mobilization, as well as an increase in the coefficient of efficiency of oxidative processes, allowing to reach the necessary level of energy supply of damaged or functionally depleted cells. In this case, the additional supply of tissues with oxygen is carried out in the absence of toxic effects of excess oxygen content, as it takes place when breathing with pure oxygen or oxygenobarotherapy.

The study of biological effects of argon has begun relatively recently and led to the discovery of facts indicating organoprotective and neuroprotective effects when exposed to oxygen-argon breathing mixtures. According to the authors of the monograph "Basics of barophysiology, diving medicine, barotherapy and inert gas treatment" (B.N. Pavlov et al edited by A.I. Grigoryev, M., Publ. Granp Poligraf, 2008, p. 378-380), the basis of these and other favorable effects of argon is its pronounced antihypoxic effect associated with facilitation of oxygen transfer to cells from the blood, as well as improved oxygen utilization, resulting in "pushing back" the threshold of terminal hypoxia and cell death, expanding the functional potential of cells, tissues and the body as a whole.

Positive effect of argon on metabolic processes in cells and tissues is confirmed by comparative studies on preservation of organs for transplantation (in particular, kidneys) in Celsior+argon, Celsior+xenon, Celsior+atmospheric air solution and subsequent heterotopic transplantation. Kidney transplantation stored in Celsior+argon medium has been shown to increase survival (7/8 vs. 3/8 pigs survived after surgery) and faster recovery of renal function (creatinine clearance, duration of tubulopathy, proportion of sodium excreted were evaluated) (see Faure A., Bruzzese L., Steinberg J.G., Jammes Y., Torrents J., Berdah S.V., Garnier E., Legris T., Loundou A., Chalopin M., Magalon G., Guieu R., Fenouillet E., Lechevallier E. Effectiveness of pure argon for renal transplant preservation in a preclinical pig model of heterotopic autotransplantation // J. Transl. Med. 2016, V. 14).

The neuroprotective effect of gas breathing media containing argon has also been described in a number of works. The studies confirm the positive effect of argon on the tolerance of hypoxic conditions; a positive effect of ischemic postconditioning with gas mixtures of 70 vol.% Ar, 30 vol.% O2 for one hour had been shown, which resulted in a decrease in cortical neuronal death and severity of neurological symptoms compared with classical resuscitation (see, for example, Brucken A., Kurnaz P., Bleilevens C., Derwall M., Weis J., Nolte K., Rossaint R., Fries M. Dose dependent neuroprotection of the noble gas after cardiac arrest in rats is not mediated by K(ATP)-channel opening // Resuscitation, 2014. V. 85, 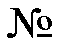 6 - pp. 826-832). At the same time, in experiments on rats and pigs, the neuroprotective properties of argon proved to be dose-dependent. At the same time, after artificially induced ischemia in the experimental subjects, the application of the above argon-containing ARGM produced a positive neurological effect.

Therefore, the use of oxygen-argon mixtures (in an approximate ratio of 65:35 or close to it) for elimination of extreme and terminal hypoxic states (in normobaric and hyperbaric variants) is the method of choice in nonpharmacological care for patients in such states. Cerebral or coronary circulatory disorders belong to such conditions.

Prospects for the use of another inert gas - xenon - to relieve acute ischemic attacks with impaired cerebral or coronary circulation and keep patients alive are due to its unique physical and chemical properties. Xenon combines low toxicity with the ability to dissolve in biological fluids and cell membranes and affect metabolic and cellular processes. First of all, xenon is used as an anesthetic and pain reliever (a mixture of oxygen and xenon), with properties approaching those of an "ideal anesthetic" (see Burov N.E. Xenon in anesthesiology. - M.: Puls, 2000. 291 p.). It helps to quickly put a patient to sleep; once anesthesia ceases, there is a quick and easy awakening without any undesirable effects, such as irritation of the respiratory tract, respiratory depression, toxic effects, etc. Medical xenon is not addictive and is eliminated from the body within minutes. Meanwhile, the achieved effect lasts for a longer time. In anesthesia practice, xenon-oxygen mixtures are used in approximate ratios of 50:50 and close to them.

At the same time, such mixtures with a lower content of xenon (10-20 vol.%) can be used in rehabilitation, sports and occupational medicine for urgent reduction of physical fatigue, relief of psycho-emotional stress, normalization of sleep, and correction of borderline functional states (see, for example, Kalmanov A.S. et al. Operational correction of functional condition of divers by inhalation of special xenon gas mixtures during training sessions // Aerospace and environmental medicine. 2016. T. 50. No. 3. P. 48-54). In this case, xenon acts as a means of artificial decrease in the activity of neurons of the higher sections of the cerebral cortex and other parts of the brain, which can be considered as a kind of "parabiotic" effect.

It is known that the parabiosis state is characterized by a significant reduction of energy and oxygen demand of the most active cells and tissues, as well as their transition to the most economical level of functioning while maintaining vitality and possibility of recovery. Therefore, the inclusion of xenon at low concentrations into the said oxygen-argon mixture for the purpose of relieving acute ischemic attacks with impaired cerebral or coronary circulation is pathogenetically sound, as in this case, the antihypoxic effects of oxygen and argon will be synergistically combined with xenon-induced significant reduction of oxygen and energy demand of vital tissues and organs and, first of all, the higher sections of CNS.

Experimental confirmation of these effects of inert gases were studies where the fact of significant prolongation of life of laboratory animals (in particular, rats) in a confined (without gas exchange) space when filling the chamber with oxygen-argon and oxygen-xenon mixtures compared with the presence of control group animals in an air environment (the initial oxygen concentration in all cases was the same) was proved. It was shown that when rats were exposed to argon medium, oxygen consumption was reduced by an average of 27% compared to the control, and the maximum survival time increased by 33%. Oxygen consumption in xenon decreased by 3.2 times, the time of maximum animal survival increased by 3.5 times (see Ananyev V.N. Effect of inert gases on oxygen uptake in an enclosed space in normobaric conditions // Materials of the IX All-Army Scientific-Practical Conference with International Participation "Barotherapy in complex treatment and rehabilitation of the wounded, sick and affected persons." - Saint-Petersburg., 2015. P. 80.).

In addition, proof of the effectiveness of using inhaled gas mixtures was obtained in experimental studies in the application of oxygen-argon-xenon mixtures to keep alive laboratory animals with acute massive blood loss of severe degree. It was found that placing rats with massive blood loss in a chamber with oxygen-argon-xenon gas environment for a period of 8 hours significantly improved animal survival: 3 out of 24 animals of the experimental groups died (mortality 12.5%). In the control group of rats with similar blood loss in a gas medium with similar nitrogen oxygen content, 5 out of 12 individuals died within 8 hours (lethality 41.7%, p<0.05).

Thus, considering the specifics of action of each of the gases described on the body, their safety and ease of use, the authors proposed an inhalation gas mixture containing xenon - 1-10 vol.%; argon - 30-35 vol.%, oxygen - 60-65 vol.% for use as a means of relieving acute ischemic attacks in premedical care and self-help. The time of application of this mixture is governed by the general therapy regimen and should be at least 20 to 40 minutes, which is enough time for the arrival of the ambulance.

The stated concentrations of gases in the artificial gas mixture (ARGM) are justified by the following arguments. Progressive growth of tissue oxygen supply due to increased oxygen diffusion from alveoli into blood takes place at normal pressure when the oxygen content in the inhaled air increases to about 60-65 vol.%. At higher concentrations of oxygen in ARGM, the rate at which it enters the body slows down due to the extremely low solubility of this gas in blood and complete oxygen saturation of circulating hemoglobin of arterial blood. In addition, when breathing pure oxygen or ARGM with more than 65 vol.%, the risk of so-called oxidative stress increases - it is a cascade of biochemical hyperperoxidation reactions, the products of which have a pronounced toxic effect on body cells, which can aggravate the damaging effects of the ischemic attack. The content of xenon inert gas in ARGM, exceeding 10-15 vol.%, can also lead to intoxication of damaged cells and, above all, of neurons of the higher brain cortex, which is especially dangerous in cerebral stroke or the risk of its development. Moreover, the introduction of a patient into xenon anesthesia at the stage of premedical care is inadmissible. As for the optimal concentration of argon in ARGM, numerous studies have proved that the optimal antihypoxic and organoprotective effect of this gas is achieved at its content in a normobaric breathing mixture in the range of 30-35 vol.%. The higher content of argon, on the one hand, is ineffective, and on the other hand, it can also be accompanied by the risk of developing toxic effects on ischemic tissues.

The management of acute ischemic attacks with impaired cerebral or coronary circulation by using argon-containing respiratory gas mixtures with an increased oxygen and xenon content is carried out as follows. A patient with primary signs and suspected stroke or heart attack is transferred to artificial gas breathing with an individual inhalation device (a mask or mouthpiece), containing xenon 1-10 vol.%, argon 30-35 vol.%, and oxygen 60-65 vol.%.

In this case, the impact on a patient with an acute ischemic attack artificial gas mixture of the specified composition is carried out mainly at the stage of premedical care before delivery to the hospital, for 20-40 minutes or more. At the same time, if necessary, exposure of the patient to ARGM of the specified composition can also be carried out during the provision of specialized medical care, for example, when transporting the patient to a hospital.

In addition, if necessary, the artificial gas mixture of the above composition can be used under hospital conditions during resuscitation of patients with an acute ischemic attack and impaired cerebral or coronary circulation.

A face mask, respirator or mouthpiece, connected to a cylinder with oxygen-argon- xenon gas mixture of the specified composition can be used as an individual inhalation device.

The physiological effects arising as a result of and in the period of exposure to these gases on the body allow "postponing" the threshold of irreversible damage to the cells and tissues of vital organs, prolonging the patient's vitality, enabling his/her transportation to a medical facility to obtain qualified care.

The effect of this invention is to significantly reduce the probability and volume of brain and myocardial damage, as well as the lethal outcome at the stage of acute ischemic attacks with impaired cerebral or coronary circulation at the premedical stage in domestic conditions.

## Claims

1. A medicinal product for use by inhalation in the emergency management of acute ischemic attacks
with impaired cerebral or coronary circulation, which is an artificial gas mixture including oxygen and argon, **characterized in that** it contains xenon 1-10 vol.%, argon 30-35 vol.%, and oxygen 60-65 vol.%.

## Patentansprüche

1. Arzneimittel zur Verwendung durch Inhalation bei der Notfallbehandlung von akuten ischämischen Anfällen mit beeinträchtigter zerebraler oder koronarer Durchblutung, das ein künstliches Gasgemisch ist, das Sauerstoff und Argon enthält, **dadurch gekennzeichnet, dass** es 1-10 Vol.-% Xenon, 30-35 Vol.-% Argon und 60-65 Vol.-% Sauerstoff enthält.

## Revendications

1. Produit médicinal destiné à être utilisé par inhalation dans le traitement d'urgence des crises ischémiques aiguës avec altération de la circulation cérébrale ou coronaire, qui est un mélange gazeux artificiel comprenant de l'oxygène et de l'argon, **caractérisé en ce qu'il** contient 1 à 10 % en volume de xénon, 30 à 35 % en volume d'argon et 60 à 65 % en volume d'oxygène.
